# EUROPEAN PATENT SPECIFICATION

(11) **EP 4 385 515 B1**
(45) Date of publication and mention of the grant of the patent: **11.11.2026**
(21) Application number: 22383227.0
(22) Date of filing: 16.12.2022
(51) Int. Cl.: A61K 38/06, A61K 8/00, A61K 35/618, A61K 38/05, A61K 38/08, A61K 38/10, A61P 17/00, C07K 5/00, C07K 7/06, C07K 7/08

(54) **COMPOSITION FOR SKIN REPIGMENTATION**
ZUSAMMENSETZUNG ZUR HAUTREPIGMENTIERUNG
COMPOSITION POUR LA REPIGMENTATION DE LA PEAU

(43) Date of publication of application: 19.06.2024
(73) Proprietor: Bella Aurora Labs, S.A., 08690 Santa Coloma de Cervelló (Barcelona) (ES); Bordignon, Matteo, 35126 Padova (IT)
(72) Inventor: Bordignon, Matteo, 35126 Padova (IT); Hernández Navarro, Sergi, Santa Coloma de Cervelló (BARCELONA) (ES); Segura Tejedor, Jordi, Santa Coloma de Cervelló (BARCELONA) (ES)
(74) Representative: Pons IP

(56) References cited:
- EP-A1- 3 936 138
- WO-A2-2012/127352
- WO-A2-2014/081845
- HERNANDEZ NAVARRO SERGI ET AL: "Efficacy of a topical formulation containing MIA (Melanoma Inhibitory Activity) - Inhibitory peptides in a case of recalcitrant vitiligo in combination with UV exposure", MEDICINE, vol. 101, no. 46, 18 November 2022 (2022-11-18), pages e31833, XP093047006, Retrieved from the Internet <URL:https://www.ncbi.nlm.nih.gov/pmc/articles/PMC9678623/pdf/medi-101-e31833.pdf> DOI: 10.1097/MD.0000000000031833
- PUBCHEM: "Melitane | C43H59N13O7 | CID 10129683 - PubChem", 25 October 2006 (2006-10-25), pages 1 - 11, XP093047441, Retrieved from the Internet <URL:https://pubchem.ncbi.nlm.nih.gov/compound/Melitane#section=InChIKey> [retrieved on 20230516]
- PAI VARADRAJVASANT ET AL: "Topical peptides as cosmeceuticals", INDIAN JOURNAL OF DERMATOLOGY, VENEREOLOGY AND LEPROLOGY, vol. 83, no. 1, 1 January 2017 (2017-01-01), IN, pages 9, XP093046802, ISSN: 0378-6323, Retrieved from the Internet <URL:https://ijdvl.com/content/126/2017/83/1/Images/ijdvl_2017_83_1_9_186500.pdf> DOI: 10.4103/0378-6323.186500
- MOWLAZADEH HAGHIGHI SAGHAR ET AL: "Replacement of Arg with Nle and modified D-Phe in the core sequence of MSHs, Ac-His-D-Phe-Arg-Trp-NH2, leads to hMC1R selectivity and pigmentation", EUROPEAN JOURNAL OF MEDICINAL CHEMISTRY, vol. 151, 1 May 2018 (2018-05-01), AMSTERDAM, NL, pages 815 - 823, XP093047464, ISSN: 0223-5234, DOI: 10.1016/j.ejmech.2018.04.021
- HIROKI OHARA ET AL: "Collagen-derived dipeptide, proline-hydroxyproline, stimulates cell proliferation and hyaluronic acid synthesis in cultured human dermal fibroblasts", THE JOURNAL OF DERMATOLOGY, vol. 37, no. 4, 1 April 2010 (2010-04-01), pages 330 - 338, XP055115921, ISSN: 0385-2407, DOI: 10.1111/j.1346-8138.2010.00827.x
- NAOKI INOUE ET AL: "Ingestion of bioactive collagen hydrolysates enhance facial skin moisture and elasticity and reduce facial ageing signs in a randomised double-blind placebo-controlled clinical study : Collagen enhances skin properties", JOURNAL OF THE SCIENCE OF FOOD AND AGRICULTURE, vol. 96, no. 12, 10 February 2016 (2016-02-10), GB, pages 4077 - 4081, XP055712224, ISSN: 0022-5142, DOI: 10.1002/jsfa.7606

## Description

### Technical field

The present invention relates to the field of skin repigmentation. The invention is defined in the claims. The references to the methods of treatment by therapy or surgery or in vivo diagnosis methods in this description are to be interpreted as references to compounds, pharmaceutical compositions and medicaments of the present invention for use in those methods

### State of the art

Depigmentation of skin is a common condition, which can affect different parts of body skin, in different degrees, and can be caused by a number of local and systemic situations.

Usually, depigmentation of the skin is linked to people with vitiligo. Vitiligo is a depigmenting skin disorder, characterized by the selective loss of melanocytes, which in turn results in the disappearance of pigmentation in the affected areas of the skin.

Vitiligo has an estimated prevalence of 0.5-2% of the population in both adults and children, worldwide. Vitiligo is considered as a disease, which should not be dismissed, and whose effects can be psychologically devastating, often with a considerable burden on daily life (Bergqvist C et al., Vitiligo: A Review, Dermatology 2020, 236, 571-592).

Non-segmental vitiligo (NSV) is the most common clinical form of the disease, wherein the patches of depigmentation are usually bilateral and with some form of symmetry.

Vitiligo most commonly involves the face, digits, dorsal hands, flexor wrists, elbows, knees, shins, dorsal ankles, armpits, inguinal area, anogenital area, umbilicus, and nipples.

Vitiligo is characterized by the loss of functional melanocytes, and multiple mechanisms might contribute to this loss, including metabolic abnormalities, oxidative stress, generation of inflammatory mediators, cell detachment and autoimmune responses (Picardo et al., Vitiligo, Nat. Rev. Dis. Primers., 2015, 1, 15011).

The common therapies available at present for the management of vitiligo include topical corticosteroids, topical calcineurin inhibitors, such as tacrolimus and pimecrolimus, photochemotherapy with psoralen plus UVA radiation or with khellin plus UVA radiation, narrowband UVB radiation, oral steroids, immunosuppressants, antioxidants, surgical melanocyte transplantation, or depigmentation of remaining pigmented islands, among others (Taieb A, et al, Guidelines for the management of vitiligo: the European Dermatology Forum consensus, Br. J. Dermatol., 2013, 168 (1), 5-19).

The protein MIA (*"Melanoma inhibitory activity"*) has been suggested as another target for vitiligo therapy. In particular, in the international patent application WO-A-2012/127352 a number of peptides and peptide derivatives which are MIA inhibitors are proposed for the prevention and treatment of vitiligo.

More recently, in the international patent application WO-A-2022/008251, it has been shown that the combination of the MIA inhibitor peptides disclosed in WO-A-2012/127352 with snail secretion provides a more effective treatment of vitiligo.

Despite the proposals available in the prior art, there is still the need for more therapeutic alternatives for the effective repigmentation of skin.

### Object of the invention

The object of the present invention is a composition comprising a MIA inhibitor peptide and at least another peptide.

Another aspect of the invention is said composition for use in the treatment of vitiligo.

Another aspect of the invention is a cosmetic, non-therapeutic use of said composition for repigmenting the skin.

### Description of the Drawings

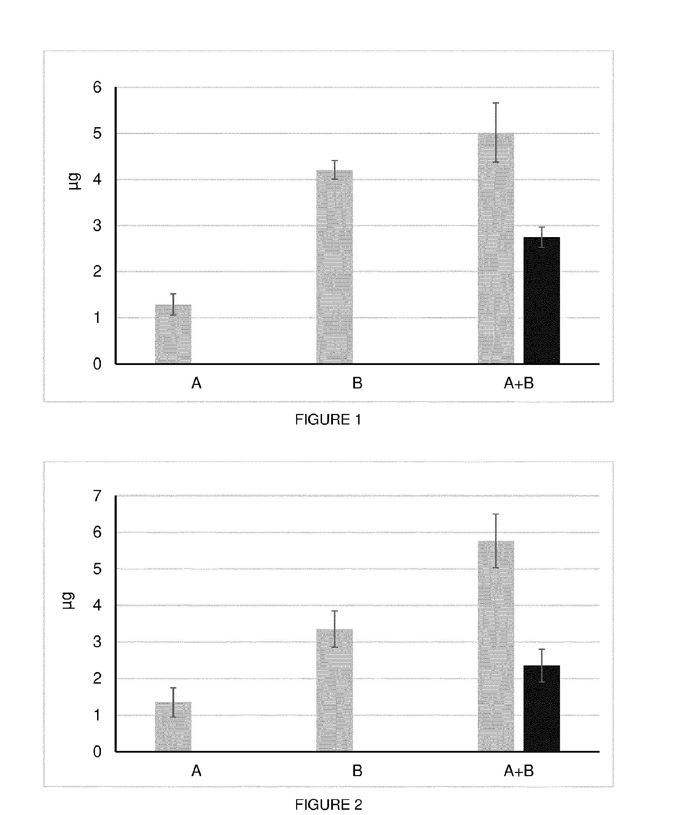

Figure 1 shows the results of the *in vitro* repigmentation assay in reconstructed human pigmented epidermis (RHPE) described in Example 1. In ordinates, the increase of the amount of melanin generated with each tested treatment versus the control is represented. In abscises, the grey columns show the results of the assay for the samples treated with the MIA inhibitor peptide of SEQ ID NO: 40 (A), with the peptide of SEQ ID NO: 50 (B) and with their combination (A+B). For comparison, the black column represents the theoretical result that was expected with the combination A+B, based on the mere addition of the effects obtained separately with A and B.
Figure 2 shows the results of the analogous assay also described in Example 1, where the tested samples were subjected to the same treatments (A, B and A+B) and were additionally irradiated.

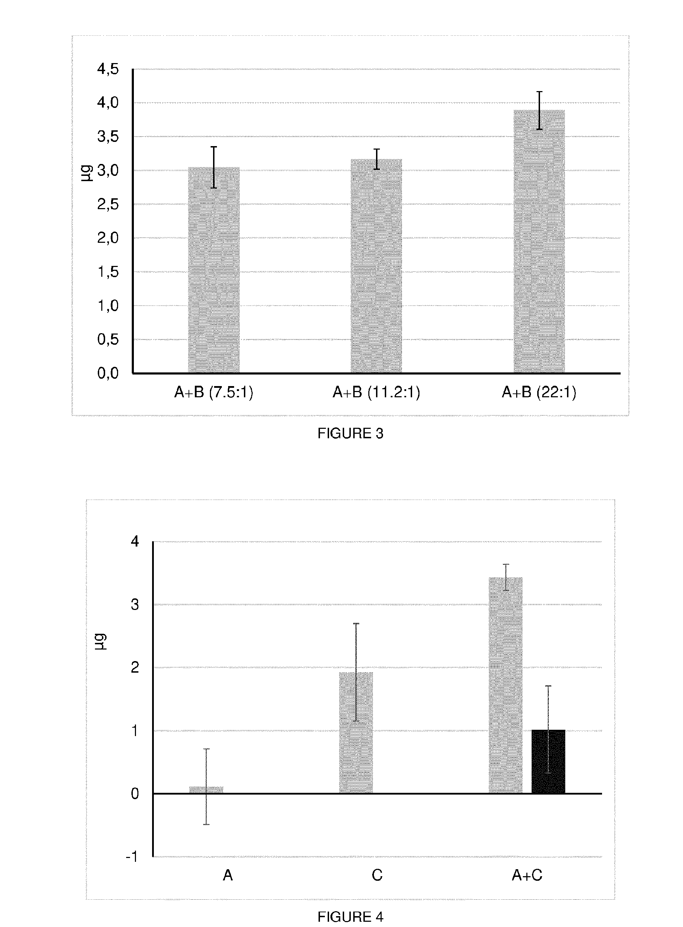

Figure 3 shows the results of the *in vitro* repigmentation assay in reconstructed human pigmented epidermis (RHPE) described in Example 3, where the samples were treated with the combination of the MIA inhibitor peptide of SEQ ID NO: 40 (A) and the peptide of SEQ ID NO: 50 (B), at different weight ratios. In ordinates, the increase of the amount of melanin generated with each tested treatment versus the control is represented. In abscises, the different tested combinations of A+B are represented. The first column represents a weight ratio A:B of 7.5:1, the second column a weight ratio A:B of 11.2:1 and the third column, a weight ratio A: B of 22:1.
Figure 4 shows the results of the *in vitro* repigmentation assay in reconstructed human pigmented epidermis (RHPE) described in Example 3. In ordinates, the increase of the amount of melanin generated with each tested treatment versus the control is represented. In abscises, the grey columns show the results of the assay for the samples treated with the MIA inhibitor peptide of SEQ ID NO: 40 (A), with the peptide of SEQ ID NO: 51 (C), and with their combination (A+C). For comparison, the black column represents the theoretical result that was expected with the combination A+C, based on the mere addition of the effects obtained separately with A and C.

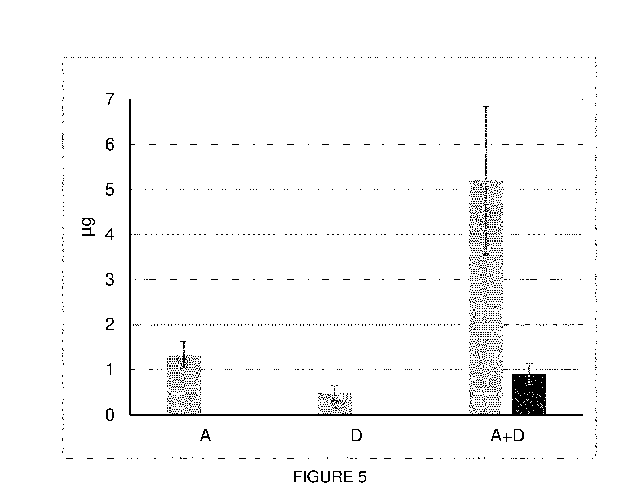

Figure 5 shows the results of the *in vitro* repigmentation assay in reconstructed human pigmented epidermis (RHPE) described in Example 4. In ordinates, the increase of the amount of melanin generated with each tested treatment versus the control is represented. In abscises, the grey columns show the results of the assay for the samples treated with the MIA inhibitor peptide of SEQ ID NO: 40 (A), with the peptide Pro-Pro (D) and with their combination (A+D). For comparison, the black column represents the theoretical result that was expected with the combination A+D, based on the mere addition of the effects obtained separately with A and D.

### Detailed description of the invention

The object of the present invention is a composition comprising:
(a) a MIA inhibitor peptide of sequence SEQ ID NO: 40;
(b) at least one additional peptide selected from the group consisting of: a peptide of SEQ ID NO: 50, a peptide of SEQ ID NO: 51 and peptide Pro-Pro; and
(c) at least one pharmaceutically acceptable excipient.

Said composition is particularly suitable for the repigmentation of skin. The authors of the present invention have found that there is a significative synergistic effect in skin repigmentation when a MIA inhibitor peptide of SEQ ID NO: 40 is combined with at least another peptide selected from those of SEQ ID NOs 50, 51 and Pro-Pro. The outstanding repigmentation effect obtained with these combinations allows for an improved therapy of vitiligo, compared to the treatments available so far in art.

In the present description, as well as in the claims, the singular expressions, generally preceded by the articles "a", "an" or "the", are meant to include also the plural forms, unless the context clearly indicates otherwise. All percentages are expressed by weight, unless specifically stated otherwise. Numeric values preceded by the term "about" are meant to include also a certain variation around such value, namely a variation or ±5% of the stated amount. Numeric ranges defined by lower and upper endpoints are meant to include also said stated endpoints and they also include any narrower sub-range.

The amino acids are represented herein either using the one-letter code or the three-letter code, as are both well-known by the skilled in the art, according to IUPAC-IUB Commission on Biochemical Nomenclature, as described in the book Biochemical Nomenclature and Related Documents, 2nd edition, Portland Press, 1992, London [ISBN 1-85578-005-4]. Following said nomenclature, the peptides of this description are represented with the N-terminus on the left side of the formula and with the C-terminus on the right side of the formula. Some specific non-proteinogenic amino acids are represented herein as follows: D-Phenylalanine, is represented as DPhe and Norleucine is represented as Nle.

### MIA inhibitor peptide

A MIA inhibitor peptide is a peptide having activity as inhibitor of the human melanoma inhibitory activity (MIA) protein, in particular, those disclosed in the international patent application WO-A-2012/127352, namely, a peptide having a sequence selected from SEQ ID NO: 1 to SEQ ID NO: 49:
- SEQ ID NO: 01 VPHIPPN
- SEQ ID NO: 02 MPPTQVS
- SEQ ID NO: 03 QMHPWPP
- SEQ ID NO: 04 QPPFWQF
- SEQ ID NO: 05 TPPQGLA
- SEQ ID NO: 06 IPPYNTL
- SEQ ID NO: 07 AVRPAPL
- SEQ ID NO: 08 GAKPHPQ
- SEQ ID NO: 09 QQLSPLP
- SEQ ID NO: 10 GPPPSPV
- SEQ ID NO: 11 LPLTPLP
- SEQ ID NO: 12 QLNVNHQARADQ
- SEQ ID NO: 13 TSASTRPELHYP
- SEQ ID NO: 14 TFLPHQMHPWPP
- SEQ ID NO: 15 VPHIPPNSMALT
- SEQ ID NO: 16 RLTLLVLIMPAP
- SEQ ID NO: 17 YNLPKVSSNLSP
- SEQ ID NO: 18 MPPTQVSKFRLI
- SEQ ID NO: 19 ANIDATPLFLRA
- SEQ ID NO: 20 LLRTTETLPMFL
- SEQ ID NO: 21 SALEPLV
- SEQ ID NO: 22 GSPTPNA
- SEQ ID NO: 23 APSHATH
- SEQ ID NO: 24 TTVGHSD
- SEQ ID NO: 25 THFSTFT
- SEQ ID NO: 26 SLLLDTS
- SEQ ID NO: 27 SVAMKAHKPLLP
- SEQ ID NO: 28 NTIPGFASKSLD
- SEQ ID NO: 29 VSNYKFYSTTSS
- SEQ ID NO: 30 VSRHQSWHPHDL
- SEQ ID NO: 31 HLNILSTLWKYR
- SEQ ID NO: 32 HNASPSWGSPVM
- SEQ ID NO: 33 SHPWNAQRELSV
- SEQ ID NO: 34 HHWPFWRTLPLS
- SEQ ID NO: 35 WHTKFLPRYLPS
- SEQ ID NO: 36 NNTSFTVVPSVP
- SEQ ID NO: 37 SHLSTWKWWQNR
- SEQ ID NO: 38 FHWHPRLWPLPS
- SEQ ID NO: 39 WHWTYGWRPPAM
- SEQ ID NO: 40 FHWRYPLPLPGQ
- SEQ ID NO: 41 WHWPLFIPNTTA
- SEQ ID NO: 42 WHNGIWWHYGVR
- SEQ ID NO: 43 HHLNYLWPWTRV
- SEQ ID NO: 44 FWHRWSTFPEQP
- SEQ ID NO: 45 WHMSYFWTRPPQ
- SEQ ID NO: 46 FHLNWPSRADYL
- SEQ ID NO: 47 WHKNTNWPWRTL
- SEQ ID NO: 48 ALSPSQSHPVRS
- SEQ ID NO: 49 GTQSTAIPAPTD
or a derivative thereof.

The MIA inhibitor peptide, according to the object of the present invention, has the sequence SEQ ID NO: 40.

A derivative of a peptide having a sequence selected from SEQ ID NO: 1 to SEQ ID NO: 49 means a peptide with some small variations around these sequences, still retaining the MIA inhibitory activity. Examples of suitable variations are described below.

One suitable derivative is a peptide having one of the above sequences wherein one additional amino acid is added or wherein one amino acid of the sequence is deleted.

Another suitable derivative is a peptide wherein one or more of the amino acids of the sequence is/are substituted by another natural or non-natural amino acid, preferably no more than 3 amino acids of the sequence are substituted, preferably no more than 2 amino acids, and more preferably only one amino acid is substituted by another natural or non-natural amino acid.

Natural amino acids, also known as proteinogenic amino acids, as is well-known in the art, are those 20 amino acids which usually occur in natural proteins and peptides.

Also as is well-known in the art, non-natural (or unnatural or unusual or non-proteinogenic) amino acids include, for example, those based on natural amino acids but wherein one or more atoms are substituted with functional groups comprising up to 50 atoms selected from C, H, N, S, O, P, F, Cl, Br, I and Se. Suitable, non-limiting, examples of such non-naturally occurring amino acids are trans-3-methylproline, 2,4-methanoproline, cis-4-hydroxyproline, trans-4-hydroxyproline, N-methylglycine, allo-threonine, N-methylthreonine, hydroxyethylcysteine, hydroxyethylhomocysteine, nitroglutamine, homoglutamine, pipecolic acid, tert-leucine, norvaline, 2-azaphenylalanine, 3-azaphenylalanine, 4-azaphenylalanine, and 4-fluorophenylalanine. Several methods are known in the art for incorporating non-naturally occurring amino acid residues into peptides or proteins.

Other suitable derivatives are those peptides of sequences 1 to 49, also including those having amino acid additions/deletions or substitutions, as discussed above, wherein at least one amino acid is modified, i.e., an additional moiety or functional group is added thereto. Such modifications may have the purpose of improving the stability, solubility and/or skin penetration of the peptide. Suitable modifications can be selected from glycosylation, acetylation (e.g., N-terminus), amidation (e.g., C-terminus), hydroxylation (e.g., hydroxyproline), carboxylation (e.g., γ-carboxyglutamate), phosphorylation, alkylation, N-terminus lipidation (i.e., adding saturated or unsaturated C₁₂-C₁₈ fatty acids, such as myristic or palmitic, at the N-terminus, through an amide bond), or prenylation. Common modifications of the peptide sequences are acetylation of the N-terminus and amidation of the C-terminus. C-terminal amidation includes the formation of terminal amido groups of formula -CONH₂, -CONHR or -CONR₂, wherein R is typically a short (e.g., C₁-C₄) alkyl group. The simplest and most common C-terminal amido group is -CO-NH₂.

In an embodiment, the MIA inhibitor peptide derivative is a peptide which is acetylated at the N-terminus and/or amidated at the C-terminus, preferably is acetylated at the N-terminus and amidated at the C-terminus.

In a preferred embodiment, the MIA inhibitor is a peptide:
- selected from SEQ ID NO: 1 to SEQ ID NO: 49, preferably selected SEQ ID NOs 12-20 and 27-49, more preferably selected from SEQ ID NOs 34, 38, 40 and 41, and still more preferably is a peptide of SEQ ID NO: 40; and wherein optionally
- at least one amino acid of the sequence is modified and wherein the modification is selected from glycosylation, acetylation, amidation (C-terminal), hydroxylation (hydroxyproline), carboxylation (gamma-carboxyglutamate), phosphorylation, alkylation, N-terminal lipidation and prenylation, preferably the sequence is acetylated at the N-terminus and/or amidated at the C-terminus, and more preferably is acetylated at the N-terminus and amidated at the C-terminus.

The term "MIA inhibitor peptide" or "MIA inhibitor", as used interchangeably herein, refer widely to the MIA inhibitor substance used in the composition, either if it is strictly a peptide sequence or a modified sequence as disclosed above.

In an embodiment, the MIA inhibitor peptide has a sequence as defined above and is not modified.

Those peptides and derivatives thereof can be prepared following standard methods in peptide synthesis, typically, by solid phase synthesis, or may be obtained commercially from several sources.

The composition of the invention may comprise one or more MIA inhibitor peptide, i.e., a single MIA inhibitor substance or the combination of two or more MIA inhibitors. When more than one MIA inhibitor is used, they may be used in any proportion and, therefore, the amount of MIA inhibitor as stated herein means the total amount of MIA inhibitor substances contained in the composition.

As disclosed in the international patent application WO-A-2012/127352 or in the international patent application WO-A-03/064457 or in the article Stoll et al., The extracellular human melanoma inhibitory activity (MIA) protein adopts an SH3 domain-like fold, EMBO J., 2001, 20(3), 340-349, the activity as MIA inhibitors of the peptides of sequences 1 to 49 was determined in a phage display screening assay using recombinant human MIA.

The derivatives of the peptides of sequences 1 to 49, as discussed above, can be tested for their MIA inhibitory activity using standard methods, for example, a ligand binding assay using recombinant human MIA, as discussed in Stoll *et al., op. cit.,* or as disclosed in Schmidt et al., Targeting melanoma metastasis and immunosuppression with a new mode of melanoma inhibitory activity (MIA) protein inhibition, 2012, PLoS ONE 7(5): e37941.

The MIA inhibitor peptide can be added as such to the composition or may be encapsulated within different polymeric materials or conjugated to different carrier substances for improving its transdermal delivery through the stratum corneum to reach the target MIA protein in the vitiliginous melanocytes, which are located in the bottom layer of the epidermis, the stratum basale, attached to the basement membrane.

Encapsulation techniques are a well-known in the art for the delivery of active ingredients.

In one embodiment, the MIA inhibitor peptide is micro-encapsulated using liposome technology.

Liposomes are commonly used in dermal formulations for improving penetration of actives through the skin layer. As is well known in the art, liposomes are spherical vesicles with sizes generally in the range between about 60 nm and 300 nm and are most often composed of phospholipids which form at least one phospholipid bilayer, but may also include other lipids. Liposomes contain hydrophilic cores in which hydrophilic actives may be encapsulated, while hydrophobic actives are incorporated in the bilayer, so liposomes are suitable carriers for both hydrophilic and lipophilic actives (Knoth et al., Nanocarrier-Based Formulations: Production and Cosmeceutic Applications, in: Cosmetic Formulation. Principles and Practice, Benson H.A.E., Roberts M.S., Rodrigues Leite-Silva V. and Walters K.A., editors, CRC Press, 2019). Liposomes may be prepared by well-known techniques; in general, the preparation methods involve mixing the membrane-forming lipids, in an organic phase, drying, subsequent hydration of the lipids and further size reduction by different mechanical treatment such as sonication, extrusion or homogenization.

In another embodiment, the MIA inhibitor is encapsulated within a microcapsule or nanocapsule made of a biodegradable polymer, as are well-known in the art, for example, as those disclosed for the delivery of the peptides of sequences 1 to 49 (WO-A-03/064457, *op.cit*.) including polyesters, polyamino acids, polyalkyl cyanoacrylates, polyphosphazenes, or polyethylene oxide. Common polymers used for encapsulation are, for example, polyvinyl alcohol (PVA), poly(lactic acid) (PLA), poly(lactic-co-glycolic acid) (PLGA) or their block co-polymers with poly(ethylene oxide) or poly(ethylene glycol). Other suitable biodegradable polymers are poly(propylene fumarate-co-ethylene glycol) [P(PF-co-EG)] block copolymer, poly-anhydride poly(fumaric-co-sebacic) anhydride, alginate, dextran, chitosan, hydroxyapatite, collagen, fibrin, hyaluronic acid, carbomers, and mixtures thereof.

In another embodiment, the MIA inhibitor peptide is conjugated with nanoparticles, namely, with lipid nanoparticles, polymer nanoparticles, magnetic nanoparticles or metallic nanoparticles, which may act as carriers to facilitate the penetration of the MIA inhibitor through the stratum corneum.

In one particular embodiment, the MIA inhibitor peptide is conjugated with gold nanoparticles, which are described to improve penetration through skin (Gupta et al., J. Phys. Chem. B, 2016, 7133-7142). Those nanoparticles have a gold core coated with the MIA inhibitor peptide. The MIA inhibitor peptide is conjugated to the gold nanoparticles, typically by means of an electrostatic ionic bond between the negative charges of the gold surface of the nanoparticles and the positive charges of amino acids of the peptide carrying amino groups in the side chain (lysine, arginine, histidine, asparagine or glutamine). The gold nanoparticles have a size of less than 1000 nm, preferably comprised between 50 and 200 nm. The conjugates may be prepared by reducing Au (III) to Au (0) with a reducing agent and treating the gold nanoparticles obtained with the MIA inhibitor peptide, for example, as disclosed in the international patent application WO-A-2019/185696.

The amount of the MIA inhibitor peptide in the composition of the invention, either if it is added in free form or encapsulated or conjugated with a carrier, as disclosed above, is typically comprised between 0.0001% and 1%, preferably comprised between 0.0005% and 0.1%, and more preferably comprised between 0.001% and 0.05%, wherein the percentages are expressed as weight of the MIA inhibitor relative to the total weight of the composition.

### Additional peptide

The additional peptide (b) that is combined with the MIA inhibitor peptide is selected from:
- a peptide of SEQ ID NO: 50,
- a peptide of SEQ ID NO: 51, and
- peptide Pro-Pro;
wherein:
- SEQ ID NO: 50 is Nle-Ala-His-DPhe-Arg-Trp
- SEQ ID NO: 51 is His-DPhe-Arg-Trp.

The peptide of SEQ IDE NO: 50 is disclosed, for example, in the international patent application WO-A-03/64458, for its melanotropic, anti-inflammatory and/or anti-allergic use.

Within the meaning of the present invention, the peptide of SEQ IDE NO: 50 may be optionally acetylated at the N-terminus (CH₃-CO-) and/or an amidated (-NH₂) at the C-terminus.

In an embodiment, the peptide of SEQ ID NO: 50 includes an acetyl group at the N-terminus and an amide group (-NH₂) at the C-terminus, and can be represented as Ac-Nle-Ala-His-DPhe-Arg-Trp-NH₂.

As shown in Example 1 (and in Figures 1 and 2) the combination of a MIA inhibitor peptide (of SEQ ID NO: 40) and the peptide of SEQ ID NO: 50 causes an outstanding increase in the production of melanin, in an *in vitro* assay performed with reconstructed human pigmented epidermis. The combination of both peptides provides a synergistic effect, as the increase in melanin production is clearly higher than that expected with a mere addition of the effects of the individual peptides.

The peptide of SEQ ID NO: 51 is a melanocortin agonist, as described, for example, in Holder et al, J. Med. Chem., 2002, 45 (13), 2801-2810.

Within the meaning of the present invention, the peptide of SEQ ID NO: 51 may include a N-terminus lipidation, i.e., the addition of a saturated or unsaturated C₁₂-C₁₈ fatty acid, such as myristic or palmitic, at the N-terminus, through an amide bond; and/or may be amidated (-NH₂) at the C-terminus. In an embodiment, the N-terminus of SEQ ID NO: 51 includes a palmitoyl group and the C-terminus an amide group (-NH₂).

As shown in Example 3 (and in Figure 4) the combination of a MIA inhibitor peptide (of SEQ ID NO: 40) and the peptide of SEQ ID NO: 51 causes an outstanding increase in the production of melanin, in an *in vitro* assay performed with reconstructed human pigmented epidermis, which is clearly higher than that expected with the mere addition of the effects of the individual peptides.

The peptide Pro-Pro is a pro-pigmenting peptide disclosed, for example, in the international patent application WO-A-2014/080376.

The peptide Pro-Pro, within the meaning of the present invention, may include a N-terminus lipidation, i.e., the addition of a saturated or unsaturated C₁₂-C₁₈ fatty acid, such as myristic or palmitic, through an amide bond; and/or the addition of an amide group (-NH₂) at the C-terminus. In an embodiment, the N-terminus of the peptide Pro-Pro includes a palmitoyl group, and it can be represented as CH₃-(CH₂)₁₄-CO-Pro-Pro (palmitoyl-prolil-proline).

As shown in Example 4 (and in Figure 5) the combination of a MIA inhibitor peptide (of SEQ ID NO: 40) and the peptide Pro-Pro causes an outstanding increase in the production of melanin, in an *in vitro* assay performed with reconstructed human pigmented epidermis, which is clearly higher than that expected with the mere addition of the effects of the individual peptides.

In an embodiment, the MIA inhibitor peptide is combined with a peptide of SEQ ID NO: 50.

In an embodiment, the MIA inhibitor peptide is combined with a peptide of SEQ ID NO: 51.

In an embodiment, the MIA inhibitor peptide is combined with peptide Pro-Pro.

In an embodiment, the MIA inhibitor peptide is combined with at least two peptides selected from the group consisting of: a peptide of SEQ ID NO: 50, a peptide of SEQ ID NO: 51 and peptide Pro-Pro.

In an embodiment, the MIA inhibitor peptide is combined with a peptide of SEQ ID NO: 50, a peptide of SEQ ID NO: 51 and peptide Pro-Pro.

The weight ratio MIA inhibitor peptide : 40(a):additional peptide (b) in the composition of the invention is comprised between 1:1 and 100:1, preferably comprised between 2:1 and 50:1, wherein the additional peptide (b) is either the peptide of SEQ ID NO: 50 or the peptide of SEQ ID NO: 51 or the peptide Pro-Pro.

When more than one additional peptide of SEQ ID NOs 50, 51 or Pro-Pro are added to the composition, the weight ratio relates to the individual weight ratio of the MIA inhibitor peptide : 40to each one of the additional peptides.

When the composition of the invention comprises a peptide of SEQ ID NO: 50, the weight ratio of the MIA inhibitor peptide to the peptide of SEQ ID NO: 50 is preferably comprised between 2:1 and 50:1, more preferably comprised between 5:1 and 25:1.

When the composition of the invention comprises a peptide of SEQ ID NO: 51, the weight ratio of the MIA inhibitor peptide to the peptide of SEQ ID NO: 51 is preferably comprised between 2:1 and 60:1, more preferably comprised between 10:1 and 50:1, and still more preferably comprised between 20:1 and 40:1.

When the composition of the invention comprises a peptide Pro-Pro, the weight ratio of the MIA inhibitor peptide to the peptide Pro-Pro is preferably comprised between 1:1 and 10:1, more preferably comprised between 2:1 and 5:1.

### Optional additional ingredients

Optionally, the composition of the invention may contain snail secretion as additional active ingredient.

Snail secretion, also known as snail slime, is a kind of mucus or bodily secretion which is produced by snails (gastropods), by glands located in the foot of the gastropod. This mucus is made of water and a complex mixture of substances, including proteoglycans, glycosaminoglycans, glycoprotein enzymes, hyaluronic acid, copper peptides, antimicrobial peptides, and metal ions; the main components are allantoin, collagen, elastin and glycolic acid (Cillia et al., Antimicrobial properties of terrestrial snail and slug mucus, J. Complement. Integr. Med., 2018, 15(3)).

This secretion may be collected and recovered from living snails, for example as disclosed in the patent US5538740 or in the US patent application US-A-2018/0064635. In general, after subjecting the gastropods to certain stress conditions, the secretion is collected, and filtered.

Snail secretion is generally in the form of a liquid, the pH is generally comprised in the range 5-8, the density is typically from about 1.0 to about 1.1 mg/ml and the dry residue is generally comprised between 0.1% and 3%, preferably comprised between 0.2% and 2%, more preferably comprised between 0.25% and 1%, and still more preferably comprised between 0.3% and 0.7%. The protein content is typically in the range 0.1-3 mg/ml.

Snail secretion is commercially available from several sources, for example, from the company Cobiosa, Spain (sold as product Poly-Helixan PF) or from the company CENTISIA Laboratorio di Fitocosmesi di Bruno Dott.ssa Laura Francesca & C. s.a.s., Italy (sold as Prodotto 580038), among others.

Different species of gastropods may be used to obtain this secretion, including, among others, *Achatina fulica, Cornu aspersum* (or *Helix aspersa* or *Cryptomphalus aspersa or Cantareus aspersus), Helix pomatia, Helix hortensis, Helix nemoralis, Helix cardidula, Helix tchthyomma, Helix fructicicola, Helix strigella, Helix fruticum, Helix bidens, Helix arbostorum, Helix rotundata, Helix aculeata, Helix pulchella, Helix personata, Helix holoserica, Helix aperta, Helix parnassia, Helix alonensis, Helix candidissima, Helix pisana,* or *Helix gualteviana.* In a particular embodiment, the snail secretion is obtained from *Cornu aspersum,* also known as *Helix aspersa, Cryptomphalus aspersa* or *Cantareus aspersus.*

The medicinal and cosmetic use of snail secretion has been disclosed in the state of the art, for example, as antimicrobial agent, for wound repair, for skin protection, or as anti-aging agent, among other uses.

The combined use of a MIA inhibitor peptide and snail secretion is disclosed in the international patent application WO-A-2022/008251.

The amount of snail secretion in the composition of the invention, if present, is typically comprised between 0.5% and 15%, preferably comprised between 1% and 10%, more preferably comprised between 2% and 8% and still more preferably is about 5%, wherein the percentages are expressed as weight of the snail secretion relative to the total weight of the composition.

### Compositions

The composition of the invention comprises at least one pharmaceutically acceptable excipient.

The term "pharmaceutically acceptable excipient" means a component of a pharmaceutical or cosmetic composition that is not an active ingredient, which is useful for preparing the composition and is generally safe and non-toxic for human pharmaceutical or cosmetic use. In particular, the composition of the invention is typically intended to be used externally, topically applied over the affected skin area, so the pharmaceutically acceptable excipient is specifically "dermatologically acceptable", i.e., it is suitable and non-toxic for use in contact with human skin tissue.

In an embodiment, the composition according to present invention is for topical administration, i.e., it is to be spread over the skin in the area to be treated, namely, in the area showing depigmentation, typically vitiliginous depigmentation, or prone to show depigmentation. Topical, dermal, or cutaneous administration are used herein interchangeably.

Any type of formulation suitable for topical administration may be used. Typically, the composition of the invention is in the form of cream, gel, cremigel, lotion, paste, foam, solution, suspension, emulsion, milk, or stick preparation, for example, which are well-known in the art. A cremigel, for example, is a type of formulation halfway between a cream and a gel: unlike a cream, a cremigel is a gelled aqueous solution or a gelled oil, but it looks opaque as a cream, usually white in colour.

Suitable carriers may be, for example, anhydrous, as mixtures of fats, waxes, animal and plant oils and solid and liquid hydrocarbons. Or the carrier may be water or an aqueous solution of hydrophilic substances. Suitably, the carrier may be in the form of an emulsion. Emulsions may be, typically, oil-in-water emulsions, water-in-oil emulsions, water-in-oil-in-water, oil-in-water-in-oil or water-in-silicone emulsions. An emulsion may generally be described as having a continuous aqueous phase (oil-in-water and water-in-oil-in-water) or a continuous oil phase (water-in-oil and oil-in-water-in-oil). The oil phase may comprise silicone oils, non-silicone oils such as paraffin hydrocarbons, fatty alcohols (e.g. stearyl alcohol, cetyl alcohol or cetostearyl alcohol), fatty acids (e.g. stearic acid, oleic acid), fatty acid esters (e.g. isopropyl myristate, isopropyl palmitate), waxes or plant oils (e.g. castor oil, canola oil, cottonseed oil, jojoba oil or arachis oil), or mixtures thereof. The aqueous phase may comprise water or a water solution of hydrophilic substances, such as alcohols (e.g. ethanol, isopropyl alcohol), polyols (e.g. glycerol, sorbitol), alpha hydroxy acids, amino acids, protein hydrolysates, simple sugars, or polysaccharides.

Emulsifiers, which are common components of emulsions, are surface-active agents (surfactants) and include non-ionic surfactants, anionic surfactants, cationic surfactants and amphoteric surfactants. Non-ionic surfactants include, among others, ethoxylated fatty alcohols, ethoxylated fatty acid esters, alkyl glucosides or alkyl oligoglucosides, ethoxylated sorbitan fatty acid esters, monoglycerol/polyglycerol fatty acid esters, ethoxylated glycerin monesters, ethoxylated polyglyceryl esters, alkyl dimethylamine oxides, or poloxamers, among others. Anionic surfactants include alkaline soaps, alkyl sulphates, alkyl ether sulphates, alkyl sulphosuccinates, alkyl phosphates, acyl sarcosinates or acyl isethionates, among others. Cationic surfactants include quaternary ammonium salts, or pyridine salts, among others. Amphoteric surfactants include imidazoline derivatives, betaines, amidobetaines and sulphobetaines.

Other common ingredients in the formulation are, for example, emollients, humectants, preservatives, viscosity controlling agents, antioxidants, pH regulators, UV filters, chelating agents, perfumes and colorants. Common emollients are, for example, paraffin hydrocarbons, silicones, fatty alcohols, fatty acids, esters of fatty acids with alcohols, triglycerides, ceramides, phospholipids and waxes. Humectants include polyhydroxy alcohols, proteins and hydroxyl acids. Common preservatives include sorbic acid and its salts, benzoic acid and its salts, parabens, imidazolidinyl urea, diazolidinyl urea, DMDM hydantoin, sodium hydroxymethylglycinate, methylchloroisothiazolinone/methylisothiazolinone, benzyl alcohol and 2-phenoxyethanol, among others. Other additives may also be added for controlling the viscosity of the formulation, for example, xanthan gum, gellan gum, acacia, carrageenans, chitosan, collagen, tragacanth, pectin, starch derivatives, carbomers, cellulose derivatives (hydroxyethylcellulose, hydroxypropylcellulose, hydroxypropyl methylcellulose, methylcellulose, ethylcellulose, or carboxymethylcellulose, for example), polyamides, glutamides, colloidal silica or waxes (e.g. beeswax or vegetable waxes), among others.

Some ingredients of the composition may act as penetration enhancers, to facilitate the penetration of the active ingredients throughout the stratum corneum. Common penetration enhancers are, for example, short chain alcohols such as ethanol or isopropyl alcohol; long chain alcohols such as decanol, hexanol, lauryl alcohol, myristyl alcohol, octanol, octyldodecanol or oleyl alcohol; cyclic amides such as azone; esters such as ethyl acetate octyl salicylate, padimate O, ethyl oleate, glyceryl monoleate, glyceryl monocaprate, glyceryl tricaprylate, isopropyl myristate, isopropyl palmitate, propylene glycol monolaurate, or propylene glycol monocaprylate; ether alcohols such as Transcutol^{®}; fatty acids such as lauric acid, linoleic acid, linolenic acid, myristic acid, oleic acid, palmitic acid, stearic acid or isostearic acid; glycols such as dipropylene glycol, propylene glycol, 1,2-butylene glycol or 1,3- butylene glycol; pyrrolidones such as N-methyl-2-pyrrolidone or 2-pyrrolidone; sulphoxides such as decylmethyl sulphoxide or dimethyl sulphoxide; anionic surfactants; cationic surfactants; non-ionic surfactants; terpenes such as eugenol D-limonene, menthol, menthone, farnesol or neridol (as disclosed, for example, in Lane M.E., Skin penetration enhancers, Int. J. Pharm., 2013, 447, 12-21; or in Haque et al., Chemical enhancer: a simplistic way to modulate barrier function of the stratum corneum, Adv. Pharm. Bull., 2018, 8(2), 169-179).

The composition of the invention has typically a slightly acidic pH, close to the physiological pH of the skin. Common acidity regulators are organic acids, including hydroxy acids and fatty acids. Some of the most common pH regulators in cosmetic emulsions are hydroxy acids such as lactic or citric acid.

One excipient can perform more than one function. The above cited ingredients, as well as many others suitable dermatological formulation excipients, are well-known to the skilled formulator of topical compositions. Such ingredients are commercially available from several companies, such as Comercial Química Massó, SA, Evonik, DuPont or Dow Corning, among others.

The preparation of the topical composition is made according to procedures well-known to the skilled in the formulation of dermatological compositions, generally involving simple steps of mixing, and optionally heating the component ingredients.

A cream, for example, can be typically prepared by warming the oily phase and the aqueous phase separately to a temperature of about 60 to 80° C, and then mixing under stirring to prepare the emulsion.

The main ingredients of the compositions and the procedures for preparing them are described, for example, in the book: Cosmetic Formulation. Principles and Practice, Benson H.A.E., Roberts M.S., Rodrigues Leite-Silva V. and Walters K.A., editors, CRC Press, 2019, or in other similar reference books. Also, regulated cosmetic substances and ingredients are disclosed in the European Commission database "Coslng" (https://ec.europa.eu/growth/tools-databases/cosing).

Typically, for example, a cream, gel, or lotion base formulation is first prepared, using standard ingredients and procedures well-known in the art, and then the MIA inhibitor peptide, the additional peptide, and snail secretion (if present) are added and thoroughly mixed, and the pH of the composition is finally adjusted.

### Use of the composition

As shown in the in vitro repigmentation assays of the examples, the combination of the MIA inhibitor peptide and the additional peptide, as defined above, is outstandingly effective for skin repigmentation.

Another aspect of the invention is, therefore, the composition of the invention for use in the prevention and/or treatment of vitiligo.

Another aspect of the present disclosure is a method for the prevention and/or treatment of vitiligo in a patient in need thereof comprising the steps of applying a therapeutically effective amount of the composition of the invention.

The composition of the invention can also be used for the cosmetic, non-therapeutic repigmentation of small areas of depigmented, whitened skin, which may not be specifically defined as vitiliginous.

Therefore, another aspect of the invention is the cosmetic, non-therapeutic use of the composition of the invention for skin repigmentation.

Another aspect of the present disclosure is a method for skin repigmentation in a subject in need thereof comprising the steps of applying an effective amount of the composition of the invention.

The cosmetic use for skin repigmentation is understood as the procedure aimed to partially or totally restore the normal pigmentation of the depigmented or hypopigmented skin patches in a human subject.

Analogously, the treatment of vitiligo is understood as the procedure aimed to improve vitiligo symptoms, namely, to partially or totally restore the normal pigmentation of the depigmented or hypopigmented skin patches in a human subject.

In one embodiment, the prevention and/or treatment of vitiligo relates to the prevention and/or treatment of non-segmental vitiligo.

The prevention of vitiligo is aimed to prevent the onset of vitiligo symptoms and is addressed to subjects which are known to be prone to suffer from vitiligo, either for having been early diagnosed, before the appearance of symptoms, for example, by detecting the presence of MIA protein in the melanocytes, or for having some known genetic predisposition to suffer from this disease. The prevention of vitiligo also includes the treatment of patients who had previously suffered from vitiligo and have recovered from the disease, i.e., who have achieved re-pigmentation of the vitiliginous depigmented skin, to prevent them from suffering again from vitiligo symptoms.

Typically, the composition of the invention is administered topically, i.e., it is spread over the vitiliginous or depigmented skin patches.

In one embodiment, the composition of the invention may be delivered transdermally, for example, via iontophoresis, ultrasound or using microneedles (Escobar-Chávez et al. Microneedles: a valuable physical enhancer to increase transdermal drug delivery, J. Clin. Pharmacol., 2011, 51(7), 964-977).

The amount of the composition to be used, considered to be "effective" for the treatment or prevention of vitiligo or for cosmetic, non-therapeutic skin repigmentation can widely vary depending on many factors, for example, the severity and progression stage of the depigmentation condition, or the specific formulation used. The amount can be easily adjusted in each case by the skilled practitioner. In general, the amount of the topical composition applied to the affected skin may range from about 0.1 mg of composition for each cm² of skin surface area (mg/cm²) to about 100 mg/cm², preferably from about 5 mg/cm² to about 10 mg/cm².

The compositions of the present invention may be applied topically once or more times daily, for example twice, three times or four times daily. The composition is typically applied by spreading it over the skin, generally, only over the affected skin area. The duration of the treatment may be adjusted according to the repigmentation evolution. Typically, the treatment may be maintained for several weeks, for example 1, 2, 3 or 4 weeks, or several months, for example, 1 to 12 months, or even more, depending on the severity of depigmentation symptoms and the evolution of the condition. The duration of the treatment can be easily adjusted by the skilled practitioner.

Advantageously, the treatment with the composition of the invention may be combined with phototherapy, for example, by simple sunlight exposure or by the use of sunbeds, to stimulate melanocyte formation and migration and thus boosting the repigmentation.

Another aspect of the present disclosure is, therefore, a method for the prevention and/or treatment of vitiligo in a patient in need thereof comprising the steps of applying a therapeutically effective amount of the composition of the invention and exposing the treated skin area to phototherapy.

The term "phototherapy" or "light therapy" as used herein includes exposure to sunlight or to radiation of specific wavelengths, for example, UV radiation, including UVA and UVB radiation. Therefore, "phototherapy" can be performed by simple exposure to sunlight, or using sunbeds, or using a radiation emitting device, in particular, a UV emitting device.

The duration of the phototherapy is not decisive, and may range, for example, from about 2 minutes to about 2 hours daily, or on alternate days, or twice or three times weekly, among other suitable options.

Vitiligo, or skin depigmentation in general, can affect any area of skin, so the composition may be applied to any area of affected (depigmented) skin. The areas of skin most commonly affected and, therefore, more in need to be treated, are hands and face, around body openings (the eyes, nostrils, mouth, umbilicus and genital regions), and within body folds such as the underarms and groin.

In one aspect the use of the composition of the invention for treating skin depigmentation in general, or vitiligo in particular, may be combined with the use of antioxidants, in particular, when the therapy is combined with phototherapy, in order to reduce the possible negative effects of the radiation exposure.

The antioxidants are typically for oral use. Suitable antioxidants are, among others, vitamin E, vitamin C, carotenoids (such as lycopene), beta-carotene, alpha-carotene, green tea extract, zinc, selenium, *Polypodium leucotomos* extract, among others, or combinations thereof.

They can be formulated as a dietary oral supplement, typically as tablets, powders or capsules, for example, using standard excipients and preparation process, known in the art.

Another aspect of the present disclosure is also a method for the prevention and/or treatment of vitiligo in a patient in need thereof comprising the steps of applying a therapeutically effective amount of the composition of the invention, exposing the treated skin area to phototherapy and administering to the patient an antioxidant.

### Examples

### Example 1: In vitro repigmentation assay in reconstructed human pigmented epidermis (RHPE) treated with the combination of a MIA inhibitor peptide and a peptide of SEQ ID 50

For assessing the repigmentation effect, a reconstructed epidermis model was used, namely, based on adding melanocytes and keratinocytes on a polycarbonate filter (SkinEthic^{®} RHPE, available from the company Episkin).

Sample tissues were treated with the test products for 6 days (from day1 to day6). After each dosing, the samples were incubated at 37°C, and melanin extraction was performed after 6 days of treatment (at day7). Untreated samples were also included in the assay as controls. An evaluation of cell viability was also performed.

After treatment, melanin extraction was performed by plunging each sample filter into 800 µl of Solvable^{®} (Perkin Elmer) and heating at 63° C overnight. The optical density of the supernatants was measured on 300 µl extract at 405 nm using synthetic melanin as reference. 6 samples were used for each treatment and the mean value was calculated.

The tested treatments were the following:

| | **Treatment** | **Concentration in culture medium** |
|---|---|---|
| **0** | Control (no treatment) | |
| **A** | MIA inhibitor of SEQ ID NO: 40 | 3.0 ppm |
| **B** | Peptide of SEQ ID NO: 50 | 0.4 ppm |
| **A+B** | MIA Inhibitor of SEQ ID NO: 40 plus Peptide of SEQ ID NO: 50 | 1.5 ppm + 0.2 ppm (7.5:1) |

The Peptide of SEQ ID NO: 50 used in this assay was acetylated at the N-terminus.

Another analogous assay, with the same treatments, was performed but simultaneously irradiating the samples. In this second assay, the samples were irradiated with 150 J/m² at 300 W/m² three times on consecutive days (on day2, day3, and day4 of the assay).

For each of the treatments the increase in the amount of melanin generated versus the control (0) was calculated. For analysing a possible synergistic effect, the experimental increase in melanin in the combined treatment (A+B) was compared to the theoretical increase to be expected if a simple additive effect were obtained.

The results are represented below (median values of the 6 samples used, SE means Standard Error):

| | Melanin (µg) | Melanin increase vs. control (µg) | SE | Theoretical (additive effect) | SE |
|---|---|---|---|---|---|
| 0 | 33.10 | -- | | | |
| A | 34.39 | 1.29 | 0.23 | -- | |
| B | 37.31 | 4.21 | 0.20 | -- | |
| A+B | 38.12 | 5.02 | 0.64 | 2.75 | 0.22 |

The results of the above table are represented in Figure 1, wherein the grey bars represent the experimental results and the black bar the theoretical result for the combination.

The results of the assay with irradiation of the samples are shown in the following table (median values of the 6 samples used):

| | Melanin (µg) | Melanin increase vs. control (µg) | SE | Theoretical (additive effect) | SE |
|---|---|---|---|---|---|
| 0 | 34.72 | -- | | | |
| A | 36.07 | 1.35 | 0.40 | -- | |
| B | 38.07 | 3.35 | 0.50 | -- | |
| A+B | 40.48 | 5.76 | 0.74 | 2.35 | 0.45 |

The results of the above table are represented in Figure 2, wherein the grey bars represent the experimental results and the black bar the theoretical result for the combination.

In both assays, either with irradiation or without irradiation, a synergistic effect in the increase of production of melanin is observed due to the combination of both peptides, because the increase obtained with the combination is clearly higher than the expected increase due to the addition of the individual effects of both peptides.

### Example 2: In vitro repigmentation assay in reconstructed human pigmented epidermis (RHPE) treated with the combination of a MIA inhibitor peptide and a peptide of SEQ ID: 50, at different ratios

Another in vitro repigmentation assay was performed using the same reconstructed epidermis model and the same procedure as disclosed in Example 1. In this case, the assay was performed without irradiation.

The treatments assayed were the combination of the same peptides as in Example 1, but using different weight ratios between them, as summarized in the following table:

| | **Treatment** | **Concentration in culture medium** |
|---|---|---|
| **0** | Control (no treatment) | |
| **A+B_1** | MIA Inhibitor of SEQ ID NO: 40 plus Peptide of SEQ ID NO: 50 | 1.5 ppm + 0.2 ppm (7.5:1) |
| **A+B_2** | MIA Inhibitor of SEQ ID NO: 40 plus Peptide of SEQ ID NO: 50 | 1.5 ppm + 0.13 ppm (11.2:1) |
| **A+B_3** | MIA Inhibitor of SEQ ID NO: 40 plus Peptide of SEQ ID NO: 50 | 1.5 ppm + 0.07 ppm (22:1) |

The results are represented below (median values of the 6 samples used):

| | Melanin (µg) | Melanin increase vs. control (µg) | SE |
|---|---|---|---|
| 0 | 24.29 | -- | |
| A+B_1 | 27.34 | 3.05 | 0.30 |
| A+B_2 | 27.46 | 3.17 | 0.15 |
| A+B_3 | 28.18 | 3.89 | 0.28 |

The results of the above table are represented in Figure 3.

### Example 3: In vitro repigmentation assay in reconstructed human pigmented epidermis (RHPE) treated with the combination of a MIA inhibitor peptide and a peptide of SEQ ID: 51

In this example, the same reconstructed epidermis model was used, namely, based on adding melanocytes and keratinocytes on a polycarbonate filter (SkinEthic^{®} RHPE, available from the company Episkin), using the same protocol disclosed in Example 1.

The tested treatments were the following:

| | **Treatment** | **Concentration in culture medium** |
|---|---|---|
| **0** | Control (no treatment) | |
| **A** | MIA inhibitor of SEQ ID NO: 40 | 3.0 ppm |
| **C** | Peptide of SEQ ID NO: 51 | 0.1 ppm |
| **A+C** | MIA Inhibitor of SEQ ID NO: 40 plus Peptide of SEQ ID NO: 51 | 1.5 ppm + 0.05 ppm (30:1) |

For each of the treatments the increase in the amount of melanin generated versus the control (0) was calculated. For analysing a possible synergistic effect, the experimental increase in melanin in the combined treatment (A+C) was compared to the theoretical increase to be expected if a simple additive effect were obtained.

The results are represented below (median values of the 6 samples used):

| | Melanin (µg) | Melanin increase vs. control (µg) | SE | Theoretical (additive effect) | SE |
|---|---|---|---|---|---|
| 0 | 36.52 | -- | | -- | |
| A | 36.63 | 0.11 | 0.60 | -- | |
| C | 38.45 | 1.93 | 0.78 | -- | |
| A+C | 39.95 | 3.43 | 0.21 | 1.02 | 0.69 |

The results of the above table are represented in Figure 4, wherein the grey bars represent the experimental results and the black bar represents the theoretical value expected for a simple additive effect.

It is again observed a synergistic effect due to the combination of both peptides in the increase of melanin production, because the increase of the combination is clearly higher than the expected increase due to the addition of the effects of both peptides.

An analogous assay with irradiation was also performed, with similar results.

### Example 4: In vitro repigmentation assay in reconstructed human pigmented epidermis (RHPE) treated with the combination of a MIA inhibitor peptide and peptide Pro-Pro

In this example, the same reconstructed epidermis model was used, namely, based on adding melanocytes and keratinocytes on a polycarbonate filter (SkinEthic^{®} RHPE, available from the company Episkin), using the same protocol disclosed in Example 1.

The tested treatments were the following:

| | **Treatment** | **Concentration in culture medium** |
|---|---|---|
| **0** | Control (no treatment) | |
| **A** | MIA inhibitor of SEQ ID NO: 40 | 3.0 ppm |
| **D** | Peptide Pro-Pro | 1.2 ppm |
| **A+D** | MIA Inhibitor of SEQ ID NO: 40 plus Peptide Pro-Pro | 1.5 ppm + 0.6 ppm (2.5:1) |

In particular, the peptide Pro-Pro used in this assay carried a palmitoyl group at the N-terminus, i.e., it was palmitoyl-prolil-proline.

For each of the treatments the increase in the amount of melanin generated versus the control (0) was calculated. For analysing a possible synergistic effect, the experimental increase in melanin in the combined treatment (A+D) was compared to the theoretical increase to be expected if a simple additive effect were obtained.

The results are represented below (median values of the 6 samples used:

| | Melanin (µg) | Melanin increase vs. control (µg) | SE | Theoretical (additive effect) | SE |
|---|---|---|---|---|---|
| 0 | 32,01 | -- | | -- | |
| A | 33,34 | 1.33 | 0.30 | -- | |
| D | 32,49 | 0.48 | 0.17 | -- | |
| A+D | 37,21 | 5.2 | 1.65 | 0.90 | 0.24 |

The results of the above table are represented in Figure 5, wherein the grey bars represent the experimental results and the black bar represents the theoretical value expected for a simple additive effect.

It is again observed a synergistic effect in the increase of melanin production due to the combination of both peptides, because the combination provides a clearly higher increase of melanin production than the expected increase due to the addition of the effects of both peptides.

### Example 5 Preparation of compositions according to the invention

Four compositions (A, B, C and D) according to the present invention were prepared using the components listed in the following table:

| **Ingredients** | | **Weight%** | | | |
|---|---|---|---|---|---|
| | | **A** | **B** | **C** | **D** |
| A1 | Emulium^{®} Mellifera MB (polyglyceryl-6 distearate, jojoba esters, polyglyceryl-3 beeswax and cetyl alcohol) | 5.0 | 5.0 | 5.0 | 5.0 |
| A2 | Isohexadecane | 2.0 | 2.0 | 2.0 | 2.0 |
| A3 | Isopropyl isostearate | 1.0 | 1.0 | 1.0 | 1.0 |
| A4 | Isononyl Isononanoate | 2.0 | 2.0 | 2.0 | 2.0 |
| A5 | Tocopheryl acetate | 0.2 | 0.2 | 0.2 | 0.2 |
| B1 | Deionized water | 70.9 | 65.9 | 70.4 | 68.5 |
| B2 | Potassium cetyl phosphate | 0.3 | 0.3 | 0.3 | 0.3 |
| B3 | Anhydrous betaine extracted from sugar beet | 2.0 | 2.0 | 2.0 | 2.0 |
| B4 | Preservatives (methylpropanediol, caprylyl glycol and phenylpropanol) | 2.0 | 2.0 | 2.0 | 2.0 |
| B5 | Phenoxyethanol | 0.5 | 0.5 | 0.5 | 0.5 |
| B6 | Disodium EDTA | 0.1 | 0.1 | 0.1 | 0.1 |
| B7 | Glycerin | 2.0 | 2.0 | 2.0 | 2.0 |
| B8 | Xanthan gum | 0.2 | 0.2 | 0.2 | 0.2 |
| C1 | SEPIPLUS^{™} 400 (Polyacrylate-13, Polyisobutene and Polysorbate 20) | 0.5 | 0.5 | 0.5 | 0.5 |
| C2 | Cyclopentasiloxane | 2.0 | 2.0 | 2.0 | 2.0 |
| C3 | Dimethicone | 1.0 | 1.0 | 1.0 | 1.0 |
| D1 | Snail secretion | - | 5.0 | 5.0 | 5.0 |
| D2 | Anti-MIA peptide (0.1 wt%) | 5.0 | 5.0 | 1.5 | 5.0 |
| D3a | Peptide of SEQ ID NO: 50 (0.02 wt%) | 3.0 | 3.0 | - | - |
| D3b | Peptide of SEQ ID NO: 51 (0.002 wt%) | - | | 2.0 | - |
| D3c | Palmitoyl-prolil-proline (0.6 wt%) | - | - | - | 0.4 |
| D4 | Perfume | 0.3 | 0.3 | 0.3 | 0.3 |
| | Total | 100.0 | 100.0 | 100.0 | 100.0 |

The peptide components of the composition (D2, D3a, D3b and D3c) were added as aqueous or aqueous/glycerin solutions comprising about 0.1 wt%, about 0.02 wt%, about 0.002 wt% and about 0.6 wt%, respectively, of said peptides.

For preparing the composition, first components A1-A6 were heated to about 70° C to 75 °C and thoroughly mixed to obtain a first liquid phase ("phase A"). Components B1-B8 were separately mixed to form a solution, which was heated to about 70° C to 75° C. Phase A was added to this solution and was emulsified in a high-shear Ultra-Turrax mixer at 8000 rpm for about 3 minutes and the emulsion was then stirred in a blade mixer at 300 rpm for about 15 minutes. The components C1-C3 were added at 60 °C, the mixture was allowed to cool down to room temperature and components D1, D2, D3a/D3b/D3c and D4 were finally added. The final pH of the composition was checked to be in the range 5.5-6.5 (or otherwise adjusted with 10% citric acid or 10% NaOH).

## Claims

1. A composition comprising:
(a) a MIA inhibitor peptide of SEQ ID NO: 40;
(b) at least one additional peptide selected from the group consisting of: a peptide of SEQ ID NO: 50, a peptide of SEQ ID NO: 51 and peptide Pro-Pro; and
(c) at least one pharmaceutically acceptable excipient.

2. - The composition according to claim 1, **characterized in that** the amount of the MIA inhibitor peptide in the composition is comprised between 0.0001% and 1%, preferably comprised between 0.0005% and 0.1%, and more preferably comprised between 0.001% and 0.05%, wherein the percentages are expressed as weight of the MIA inhibitor peptide relative to the total weight of the composition.

3. - The composition according to claims 1 or 2, **characterized in that** it comprises a peptide of SEQ ID NO:50.

4. - The composition according to any one of claims 1 to 3, **characterized in that** it comprises a peptide of SEQ ID NO:51.

5. The composition according to any one of claims 1 to 4, **characterized in that** it comprises peptide Pro-Pro.

6. - The composition according to any one of claims 1 to 5, **characterized in that** the weight ratio of the MIA inhibitor peptide (a) to the additional peptide (b) is comprised between 1:1 and 100:1.

7. - The composition according to claim 6, **characterized in that** the weight ratio of the MIA inhibitor peptide (a) to the additional peptide (b) is comprised between 2:1 and 50:1.

8. - The composition according to any one of claims 1 to 7, **characterized in that** the composition comprises snail secretion.

9. - The composition according to claim 8, **characterized in that** the amount of snail secretion in the composition is comprised between 0.5% and 15%, preferably comprised between 1% and 10%, more preferably comprised between 2% and 8% and still more preferably is about 5%, wherein the percentages are expressed as weight of the snail secretion relative to the total weight of the composition.

10. - The composition according to any one of claims 1 to 9, **characterized in that** it is for topical administration.

11. - The composition according to claim 10, **characterized in that** it is selected from cream, gel, cremigel, lotion, paste, foam, solution, suspension, emulsion, milk, and stick.

12. - The composition according to claim 11, **characterized in that** it is selected from cream, gel and cremigel, and preferably is selected from cream and gel.

13. - The composition according to any one of claims 1 to 12 for use in the prevention and/or treatment of vitiligo.

14. - The composition for use according to claim 13, **characterized in that** vitiligo is non-segmental vitiligo.

15. - Cosmetic, non-therapeutic use of the composition according to any one of claims 1 to 12 for skin repigmentation.

## Patentansprüche

1. Zusammensetzung, umfassend:
(a) ein MIA-Inhibitorpeptid der SEQ ID NO:40;
(b) mindestens ein zusätzliches Peptid, ausgewählt aus der Gruppe bestehend aus: einem Peptid der SEQ ID NO:50, einem Peptid von SEQ ID NO:51 und Peptid Pro-Pro; und
(c) mindestens einen pharmazeutisch verträglichen Hilfsstoff.

2. Zusammensetzung nach Anspruch 1, **dadurch gekennzeichnet, dass** die Menge des MIA-Inhibitorpeptids in der Zusammensetzung zwischen 0,0001 % und 1 % umfasst, vorzugsweise zwischen 0,0005 % und 0,1 % umfasst, und noch bevorzugter zwischen 0,001 % und 0,05 % umfasst, wobei die Prozentsätze als Gewicht des MIA-Inhibitorpeptids bezogen auf das Gesamtgewicht der Zusammensetzung ausgedrückt sind.

3. Zusammensetzung nach Anspruch 1 oder 2, **dadurch gekennzeichnet, dass** sie ein Peptid der SEQ ID NO:50 umfasst.

4. Zusammensetzung nach einem der Ansprüche 1 bis 3, **dadurch gekennzeichnet, dass** sie ein Peptid der SEQ ID NO:51 umfasst.

5. Zusammensetzung nach einem der Ansprüche 1 bis 4, **dadurch gekennzeichnet, dass** sie das Peptid Pro-Pro umfasst.

6. Zusammensetzung nach einem der Ansprüche 1 bis 5, **dadurch gekennzeichnet, dass** das Gewichtsverhältnis des MIA-Inhibitorpeptids (a) zum zusätzlichen Peptid (b) zwischen 1:1 und 100:1 liegt.

7. Zusammensetzung nach Anspruch 6, **dadurch gekennzeichnet, dass** das Gewichtsverhältnis des MIA-Inhibitorpeptids (a) zu dem zusätzlichen Peptid (b) zwischen 2:1 und 50:1 liegt.

8. Zusammensetzung nach einem der Ansprüche 1 bis 7, **dadurch gekennzeichnet, dass** die Zusammensetzung Schneckensekret umfasst.

9. Zusammensetzung nach Anspruch 8, **dadurch gekennzeichnet, dass** die Menge an Schneckensekret in der Zusammensetzung zwischen 0,5 % und 15 % umfasst, vorzugsweise zwischen 1 % und 10 % umfasst, bevorzugter zwischen 2 % und 8 % umfasst und noch bevorzugter etwa 5 % beträgt, wobei die Prozentsätze als Gewicht des Schneckensekrets im Verhältnis zum Gesamtgewicht der Zusammensetzung ausgedrückt sind.

10. Zusammensetzung nach einem der Ansprüche 1 bis 9, **dadurch gekennzeichnet, dass** sie zur topischen Verabreichung bestimmt ist.

11. Zusammensetzung nach Anspruch 10, **dadurch gekennzeichnet, dass** sie ausgewählt ist aus Creme, Gel, Cremigel, Lotion, Paste, Schaum, Lösung, Suspension, Emulsion, Milch und Stift.

12. Zusammensetzung nach Anspruch 11, **dadurch gekennzeichnet, dass** sie aus Creme, Gel und Cremigel ausgewählt ist und vorzugsweise aus Creme und Gel ausgewählt ist.

13. Zusammensetzung nach einem der Ansprüche 1 bis 12 zur Verwendung bei der Vorbeugung und/oder Behandlung von Vitiligo.

14. Zusammensetzung zur Verwendung nach Anspruch 13, **dadurch gekennzeichnet, dass** es sich bei der Vitiligo um nicht segmentale Vitiligo handelt.

15. Kosmetische, nicht-therapeutische Verwendung der Zusammensetzung nach einem der Ansprüche 1 bis 12 zur Repigmentierung der Haut.

## Revendications

1. Composition comprenant :
(a) un peptide inhibiteur de la MIA de SEQ ID NO: 40 ;
(b) au moins un peptide supplémentaire choisi dans le groupe constitué de : un peptide de SEQ ID NO: 50, un peptide de SEQ ID NO: 51 et un peptide Pro-Pro ; et
(c) au moins un excipient pharmaceutiquement acceptable.

2. Composition selon la revendication 1, **caractérisée en ce que** la quantité du peptide inhibiteur de la MIA dans la composition est comprise entre 0,0001 % et 1 %, de préférence comprise entre 0,0005 % et 0,1 %, et plus préférablement comprise entre 0,001 % et 0,05 %, dans laquelle les pourcentages sont exprimés en poids du peptide inhibiteur de la MIA par rapport au poids total de la composition.

3. Composition selon les revendications 1 ou 2, **caractérisée en ce qu'**elle comprend un peptide de SEQ ID NO: 50.

4. Composition selon l'une quelconque des revendications 1 à 3, **caractérisée en ce qu'**elle comprend un peptide de SEQ ID NO: 51.

5. Composition selon l'une quelconque des revendications 1 à 4, **caractérisée en ce qu'**elle comprend le peptide Pro-Pro.

6. Composition selon l'une quelconque des revendications 1 à 5, **caractérisée en ce que** le rapport pondéral entre le peptide inhibiteur de la MIA (a) et le peptide supplémentaire (b) est compris entre 1:1 et 100:1.

7. Composition selon la revendication 6, **caractérisée en ce que** le rapport pondéral entre le peptide inhibiteur de la MIA (a) et le peptide supplémentaire (b) est compris entre 2:1 et 50:1.

8. Composition selon l'une quelconque des revendications 1 à 7, **caractérisée en ce que** la composition comprend une sécrétion d'escargot.

9. Composition selon la revendication 8, **caractérisée en ce que** la quantité de sécrétion d'escargot dans la composition est comprise entre 0,5 % et 15 %, de préférence comprise entre 1 % et 10 %, plus préférablement comprise entre 2 % et 8 % et encore plus préférablement est d'environ 5 %, dans laquelle les pourcentages sont exprimés en poids de la sécrétion d'escargot par rapport au poids total de la composition.

10. Composition selon l'une quelconque des revendications 1 à 9, **caractérisée en ce qu'**elle est destinée à une administration topique.

11. Composition selon la revendication 10, **caractérisée en ce qu'**elle est choisie parmi une crème, un gel, un crémigel, une lotion, une pâte, une mousse, une solution, une suspension, une émulsion, un lait, et un bâtonnet.

12. Composition selon la revendication 11, **caractérisée en ce qu'**elle est choisie parmi une crème, un gel et un crémigel, et de préférence est choisie parmi une crème et un gel.

13. Composition selon l'une quelconque des revendications 1 à 12 destinée à être utilisée dans la prévention et/ou le traitement du vitiligo.

14. Composition destinée à être utilisée selon la revendication 13, **caractérisée en ce que** le vitiligo est un vitiligo non segmentaire.

15. Utilisation cosmétique non thérapeutique de la composition selon l'une quelconque des revendications 1 à 12 pour la repigmentation de la peau.
